(19) Europäisches Patentamt
European Patent Office
Office européen des brevets

(11) Veröffentlichungsnummer: **0 004 022**
**B1**

(12) # EUROPÄISCHE PATENTSCHRIFT

(45) Veröffentlichungstag der Patentschrift: **16.09.81**

(51) Int. Cl.³: **C 07 C 69/74,** C 07 C 67/10, A 01 N 53/00

(21) Anmeldenummer: **79100577.0**

(22) Anmeldetag: **26.02.79**

(54) **Pentafluorbenzyloxycarbonylderivate, Verfahren zu ihrer Herstellung und ihre Verwendung als insektizide und Akarizide.**

(30) Priorität: **11.03.78 DE 2810634**

(43) Veröffentlichungstag der Anmeldung: **19.09.79 Patentblatt 79/19**

(45) Bekanntmachung des Hinweises auf die Patenterteilung: **16.09.81 Patentblatt 81/37**

(84) Benannte Vertragsstaaten: **BE CH DE FR GB IT NL**

(56) Entgegenhaltungen: **FR-A-2 271 196**

(73) Patentinhaber: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Naumann, Klaus, Dr., Wolfskaul 2, D-5000 Köln 80 (DE)**
Erfinder: **Behrenz, Wolfgang, Dr., Untergruendemich 14, D-5063 Overath (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

**EP 0 004 022 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Pentafluorbenzyloxycarbonylderivate, Verfahren zu ihrer Herstellung und ihre Verwendung als Insektizide und Akarizide.

Die Erfindung betrifft neue Pentafluorbenzyloxycarbonylderivate, Verfahren zu ihrer Herstellung und ihre Verwendung als Insektizide und Akarizide.

Es ist bereits bekannt, dass Ester der Chrysanthemumsäure, wie z. B. Chrysanthemumsäure-2,3,4,5-tetrahydrophthalimidomethylester, insektizide Eigenschaften aufweisen (vgl. Agric. Biol. Chem. 28 (1964), 914). Aus der Französischen Patentschrift Nr. 2 271 196 ist Pentachlorobenzyl-2,2-dimethyl-3-cyclobutyliden-methylcyclopropancarboxylat und seine insektizide Wirkung bekannt.

Die Wirkung dieser Verbindungen ist jedoch, insbesondere bei niedrigen Aufwandmengen, nicht immer ganz befriedigend.

Es wurden die neuen Pentafluorbenzyloxycarbonylderivate der Formel

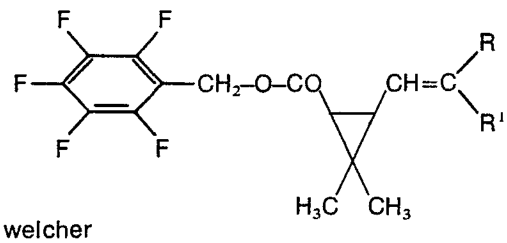

(I)

in welcher

R für Wasserstoff oder Halogen steht und

$R^1$ für gegebenenfalls substituiertes Phenyl steht, oder

worin R und $R^1$ gemeinsam für eine Alkylenkette mit mindestens zwei Kohlenstoffatomen stehen,

gefunden.

Die allgemeine Formel (I) schliesst dabei die verschiedenen möglichen Stereoisomeren, die optischen Isomeren und Mischungen dieser Komponenten ein.

Die neuen Verbindungen zeichnen sich durch eine starke insektizide und akarizide Wirksamkeit aus.

Weiterhin wurde gefunden, dass die neuen Pentafluorbenzyloxycarbonylderivate der Formel (I) erhalten werden, wenn man Carbonsäuren der Formel

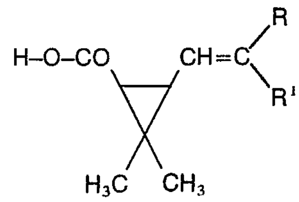

in welcher

R und $R^1$ die oben angegebene Bedeutung haben,

oder deren Salze mit Pentafluorbenzylhalogeniden gegebenenfalls in Gegenwart eines Säureakzeptors und gegebenenfalls in Gegenwart eines inerten Lösungs- oder Verdünnungsmittels umsetzt.

Überraschenderweise zeigen die erfindungsgemässen Pentafluorbenzyloxycarbonylderivate eine bessere insektizide und akarizide Wirkung als die entsprechenden vorbekannten Produkte analoger Konstitution und gleicher Wirkungsrichtung. Die Produkte gemäss vorliegender Erfindung stellen somit eine echte Bereicherung der Technik dar.

Verwendet man beispielsweise das Natriumsalz von 2,2-Dimethyl-3-cyclopropylidenmethylcyclopropancarbonsäure und Pentafluorbenzylbromid als Ausgangsverbindungen, so kann der Reaktionsverlauf durch das folgende Formelschema wiedergegeben werden:

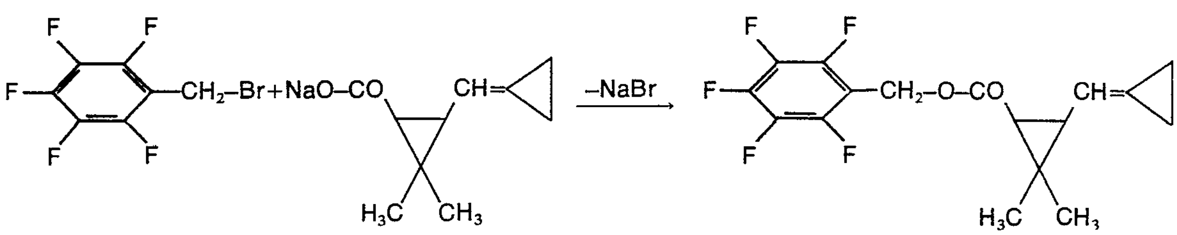


Die als Ausgangsstoffe zu verwendenden Cyclopropancarbonsäuren sind durch die Formel (II) definiert. Vorzugsweise stehen darin jedoch

R für Wasserstoff, Chlor oder Brom und

$R^1$ für Phenyl, Halogenphenyl – insbesondere Chlorphenyl und Fluorphenyl sowie für Alkylphenyl, wobei der Alkylrest 1 bis 6, insbesondere 1 bis 4 Kohlenstoffatome enthält, oder

R und $R^1$ stehen gemeinsam für Dimethylen, Trimethylen, Tetramethylen, Pentamethylen oder Hexamethylen.

Als Salze kommen bevorzugt die Alkalimetallsalze in Frage. Die Verbindungen der Formel (II) sowie ihre Salze sind zum Teil bekannt und können nach literaturbekannten Verfahren hergestellt werden (vergleiche Tetrahedron Lett. 1976, S. 4359–4362 oder FR-PS 2 067 854). Die teilweise bekannten Äthylester können nach literaturbekannten Verfahren, beispielsweise aus 3-Formyl-2,2-dimethyl-cyclopropancarbonsäureäthylester und 0,0-Dimethyl-methanphosphonsäurediester-derivaten nach folgendem Formelschema hergestellt werden:

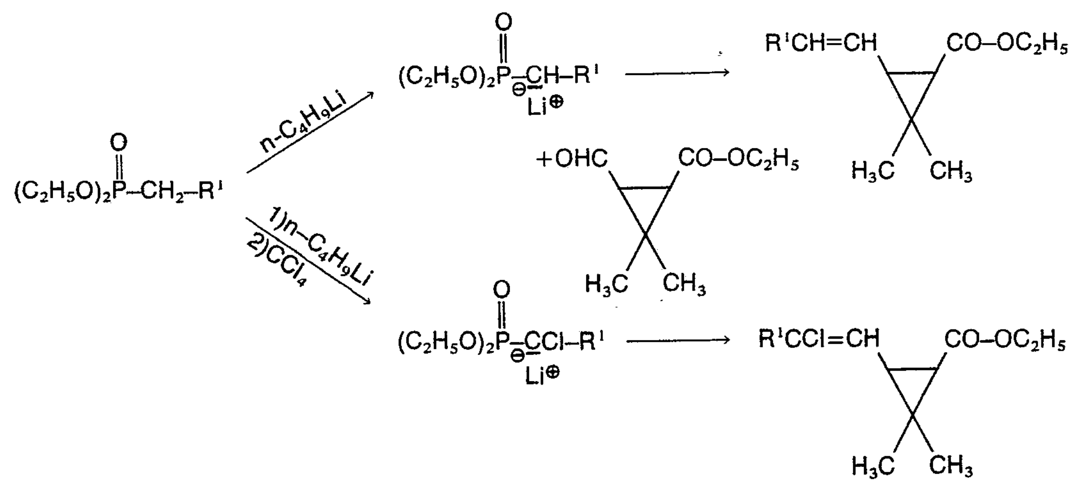


Als Beispiele für die Cyclopropancarbonsäuren bzw. deren Salze der Formel (II) seien im einzelnen genannt: 3-(2-Phenyl-vinyl)-, 3-(2-(3-Chlorphenyl)-vinyl)-, 3-(2-(4-Chlorphenyl)-vinyl)-,3-(2-(3,4-Dichlorphenylvinyl)-, 3-(2-(4-Fluorphenyl)-vinyl)-, 3-(2-(4-Methylphenyl)-vinyl)-. 3-(2-(4-Äthylphenyl)-vinyl)-, 3-(2-(4-n-Propylphenyl)-vinyl)-, 3-(2-(4-iso-Propylphenyl)-vonyl)-2,2-dimethyl-cyclopropancarbonsäure bzw. deren Natrium- oder Kaliumsalz, ferner

3-(2-Phenyl-2-chlor-vinyl)-, 3-(2-(3-Chlorphenyl)-2-chlor-vinyl)-, 3-(2-(4-Chlorphenyl)-2-chlor-vinyl)-, 3-(2-(3,4-Dichlor-phenyl)-2-chlor-vinyl)-, 3-(2-(4-Fluorphenyl)-2-chlor-vinyl)-, 3-(2-(4-Methylphenyl)-2-chlor-vinyl)-, 3-(2-(4-Äthylphenyl)-2-chlor-vinyl)-, 3-(2-(4-n-Propylphenyl)-2-chlor-vinyl)-, 3-(3-(4-iso-Propylphenyl)-2-chlor-vinyl)-2,2-dimethyl-cyclopropancarbonsäure bzw. deren Natrium- oder Kaliumsalz, ferner

3-(2-Phenyl-2-brom-vinyl)-, 3-(2-(3-Chlorphenyl)-2-brom-vinyl)-, 3-(2-(4-Chlorphenyl)-2-brom-vinyl)-, 3-(2-(3,4-Dichlorphenyl)-2-brom-vinyl)-, 3-(2-(4-Fluorphenyl)-2-brom-vinyl)-, 3-(2-(4-Methylphenyl)-2-bromvinyl)-, 3-(2-(4-Äthylphenyl)-2-brom-vinyl)-, 3-(2-(4-n-Propylphenyl)-2-brom-vinyl)-, 3-(2-(4-iso-Propylphenyl)-2-brom-vinyl)-2,2-dimethyl-cyclopropancarbonsäure bzw. deren Natrium- oder Kaliumsalz, ferner 3-Cyclopropylidenmethyl-, 3-Cyclobutylidenmethyl-, 3-Cyclopentylidenmethyl-, 3-Cyclohexylidenmethyl- und 3-Cycloheptylidenmethyl-2,2-dimethyl-cyclopropancarbonsäure bzw. deren Natrium- oder Kaliumsalz.

Das weiterhin als Ausgangsprodukt verwendete Pentafluorbenzylbromid ist bekannt und kann durch Umsetzung von Pentafluorbenzylalkohol mit Bromwasserstoffsäure hergestellt werden (J. Chem. Soc. 1961, 808–817).

Das Verfahren zur Herstellung der erfindungsgemässen Verbindungen wird bevorzugt unter Verwendung geeigneter Lösungs- oder Verdünnungsmittel durchgeführt. Als solche kommen praktisch alle inerten organischen Solventien in Frage. Hierzu gehören insbesondere aprotisch dipolare Lösungsmittel wie z.B. Aceton, Methyläthyl-, Methylisopropyl- und Methylisobutylketon, Acetonitril und Propionitril, Nitromethan, Dimethylformamid, und Dimethylacetamid, Dimethylsulfoxid, Tetramethylensulfon, N-Methylpyrrolidon und Hexamethylphosphorsäuretriamid.

Die Reaktionstemperatur kann innerhalb eines grösseren Bereichs variiert werden. Im allgemeinen arbeitet man zwischen 20 und 200 °C, vorzugsweise bei 50 bis 150 °C.

Die Umsetzung lässt man im allgemeinen bei Normaldruck ablaufen.

Zur Durchführung des erfindungsgemässen Verfahrens setzt man auf 1 Mol Cyclopropancarbonsäurederivat zwischen 0,5 und 1,5 Mol Pentafluorbenzylhalogenid oder dessen Derivat ein. Die Reaktionskomponenten werden meist in einem der angegebenen Lösungsmittel zusammengegeben und meist bei erhöhter Temperatur eine oder mehrere Stunden gerührt. Nach beendeter Reaktion wird das Lösungsmittel abdestilliert oder die Reaktionsmischung in Wasser gegossen. Das Produkt wird jeweils mit einem organischen Lösungsmittel, z.B. mit Methylenchlorid, extrahiert; die organische Phase wird dann wie üblich durch Waschen mit Wasser, Trocknen und Abdestillieren des Lösungsmittels aufgearbeitet.

Die neuen Verbindungen fallen in Form von Ölen an, die sich zum Teil nicht unzersetzt destillieren lassen, jedoch durch sogenanntes «Andestillieren», d.h. durch längeres Erhitzen unter vermindertem Druck auf mässig erhöhte Temperaturen, von den letzten flüchtigen Anteilen befreit und auf diese Weise gereinigt werden. Zu ihrer Charakterisierung dient der Brechungsindex.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophaus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips fermoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bepalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweisshydrolysate; als Dispergiermittel kommen in Frage: z. B. Lignin-Sulfitablaugen und Methylcellulose.

Es können den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem veterinärmedizinischen Gebiet.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

Beispiel A

$LD_{100}$-Test

Testtiere: Sitophilus granarius

Zahl der Testtiere: 20

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschliessend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100%, dass alle Testtiere abgetötet wurden; 0% bedeutet, dass keine Testtiere abgetötet wurden.

Bei diesem Test zeigen z. B. die folgenden Verbindungen der Herstellungsbeispiele überlegene Wirkung gegenüber dem Stand der Technik: 1,2.

Beispiel B

Mückenlarven-Test

Testtiere: Aëdes aegypti, 4. Larven

Lösungsmittel: 99 Gewichtsteile Aceton

Emulgator: 1 Gewichtsteil Benzylhydroxydiphenylpolyglykoläther

Zur Herstellung einer zweckmässigen Wirkstoffzubereitung löst man 2 Gewichtsteile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wässrigen Wirkstoffzubereitungen der gewünschten Konzentration in Gläser und setzt anschliessend etwa 25 Mückenlarven in jedes Glas ein.

Nach 24 Stunden wird der Abtötungsgrad in % bestimmt. Dabei bedeutet 100%, dass alle Larven abgetötet worden sind. 0% bedeutet, dass überhaupt keine Larven abgetötet worden sind.

Bei diesem Test zeigen z. B. die folgenden Verbindungen der Herstellungsbeispiele überlegene Wirkung gegenüber dem Stand der Technik: 1.

Beispiel C

$LT_{100}$-Test für Dipteren

Testtiere: Musca domestica (gegen P-ester und Carbamate resistent)

Zahl der Testtiere: 20

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschliessend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z.B. die folgenden Verbindungen der Herstellungsbeispiele überlegene Wirkung gegenüber dem Stand der Technik: 1,2.

Beispiel D

Laphygma-Test

Lösungsmittel 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Lyphygma frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z.B. die folgenden Verbindungen der Hersellungsbeispiele überlegene Wirksamkeit gegenüber dem Stand der Technik: 1,2.

Beispiel E

Tetranychus-Test (resistent)

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Spinnbilben abgetötet wurden; 0% bedeutet, dass keine Spinnmilben abgetötet wurden.

Bei diesem Test zeigen z.B. die folgenden Verbindungen der Herstellungsbeispiele überlegene Wirksamkeit gegenüber dem Stand der Technik: 1,2.

Beispiel F

Grenzkonzentrations-Test/Bodeninsekten

Testinsekt: Tenebrio molitor-Larven im Boden

Lösungsmittel: 3 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/1) angegeben wird. Man füllt den Boden in Töpfe und lässt diese bei Raumtemperatur stehen.

Nach 24 Stunden werden die Testtiere in den behandelten Boden gegeben und nach weiteren 2 bis 7 Tagen wird der Wirkungsrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1,2.

Beispiel G

Test mit parasitierenden adulten Rinderzecken (Boophilus microplus res.)

Lösungsmittel: Alkylarylpolyglykoläther

Zur Herstellung einer zweckmässigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (b. microplus res.) werden 1 Minute in die zu testende Wirkstoffzubereitung getaucht. Nach Überführung in Plastikbecher und Aufbewahrung in einem klimatisierten Raum wird der Abtötungsgrad bestimmt.

Bei diesem Test zeigen z. B. die folgenden Verbindungen der Herstellungsbeispiele überlegene Wirkung gegenüber dem Stand der Technik: 1,2.

## Herstellungsbeispiele

### Beispiel 1:

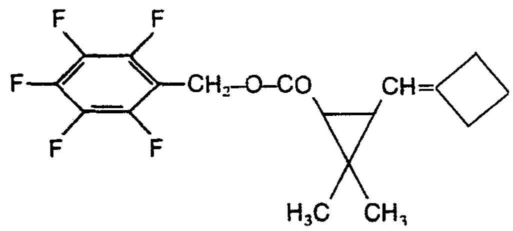

3,6 g (0,0165 Mol) 2,2-Dimethyl-3-cyclobutylidenmethylcyclopropancarbonsäure-Kaliumsalz werden in 50 ml Dimethylformamid gelöst und zusammen mit 4,3 g (0,0165 Mol) Pentafluorbenzylbromid 3 Stunden auf 110 °C erhitzt. Nach beendeter Reaktion wird das Reaktionsgemisch in 150 ml Wasser gegossen und 2 mal mit je 100 ml Methylenchlorid extrahiert. Anschliessend wird die organische Phase zweimal mit je 100 ml Wasser ausgeschüttelt und dann über Natriumsulfat getrocknet. Das Lösungsmittel wird im Vakuum abgezogen und letzte Lösungsmittelreste durch kurzes Andestillieren bei 60 °C/1 Torr Badtemperatur entfernt. Man erhält 4,0 g (67,1% der Theorie) Pentafluorbenzyl-2,2-dimethyl-3-cyclobutylidenmethylcyclopropancarboxylat als braunes Öl mit dem Brechungsindex $n_D^{30}$: 1,4850.

### Beispiel 2:

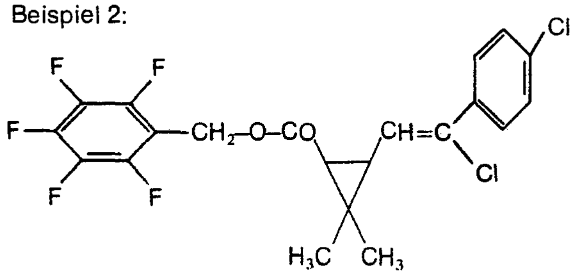

10,7 g (0,033 Mol) 2,2-Dimethyl-3-(2-chlor-2-p-chlorphenyl-vinyl)-cyclopropancarbonsäure-Kaliumsalz werden in 100 ml Dimethylformamid gelöst und zusammen mit 6,8 g (0,026 Mol) Pentafluorbenzylbromid 3 Stunden auf 120 °C erhitzt. Nach beendeter Reaktion wird das Dimethylformamid im Vakuum abdestilliert und der verbleibende Rückstand in 200 ml Methylenchlorid aufgenommen. Anschliessend wird zweimal mit je 100 ml Wasser ausgeschüttelt, die organische Phase über Natriumsulfat getrocknet und das Lösungsmittel im Vakuum abgezogen. Letzte Lösungsmittelreste werden durch kurzes Andestillieren bei 60 °C/1 Torr Badtemperatur entfernt. Man erhält 9,0 g (74,4% der Theorie) Pentafluorbenzyl-2,2-dimethyl-3-(2-chlor-2-p-chlor-phenyl-vinyl)-cyclopropancarboxylat als gelbes Öl mit dem Brechungsindex $n_D^{25}$: 1,5382.

Analog einem der Beispiele 1 oder 2 können die folgenden Verbindungen hergestellt werden:

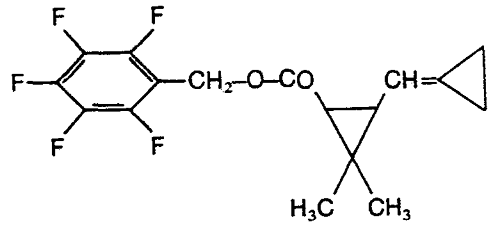

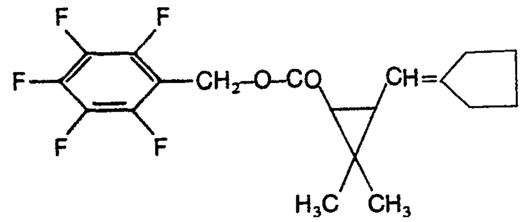

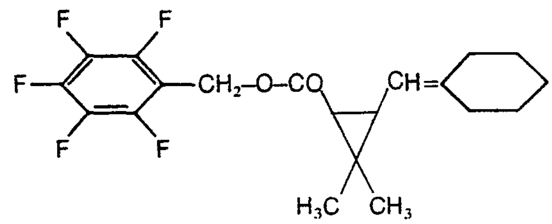

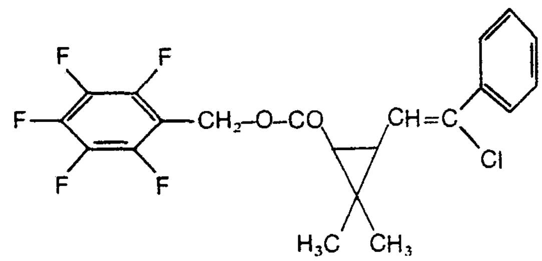

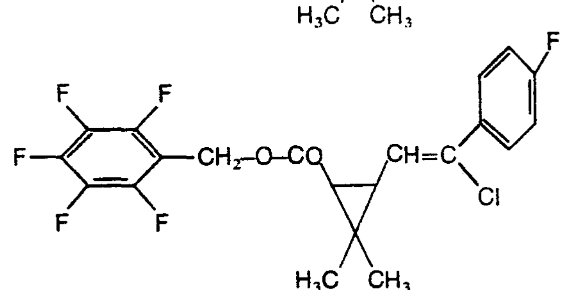

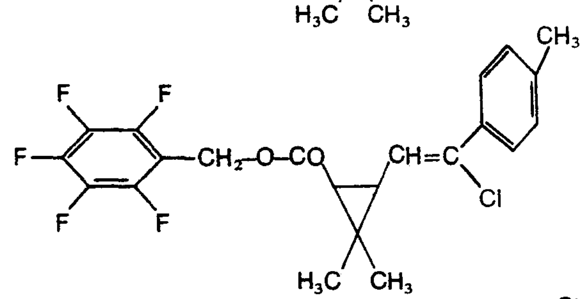

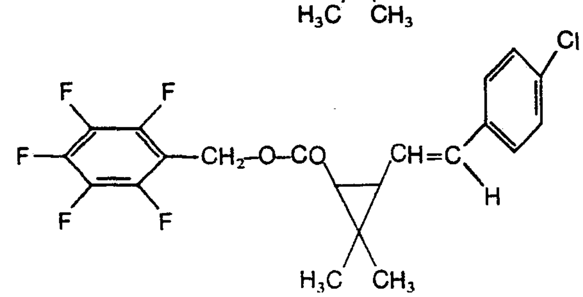

Die als Ausgangsverbindungen benötigten Cyclopropancarbonsäuren bzw. deren Salze oder Derivate können wie im folgenden beschrieben hergestellt werden:

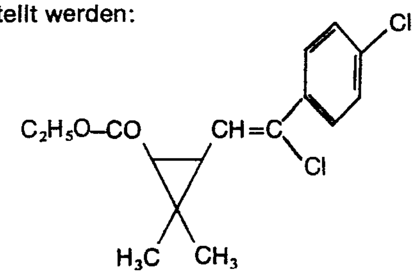

26,3 g (0,1 Mol) 4-Chlorbenzyl-phosphonsäurediäthylester werden in 400 ml absolutem Tetrahydrofuran gelöst und auf –70 °C abgekühlt. Im Stickstoffgegenstrom und unter gutem Rühren werden 0,11 Mol n-Butyllithium (15%ige Lösung in Hexan) zugetropft und dann die Reaktionsmischung noch 15 Minuten bei –70 °C nachgeführt. Anschliessend werden weiterhin unter Stickstoff 15,4 g (0,1 Mol) Tetrachlorkohlenstoff bei –70 °C zugetropft, die Reaktionsmischung färbt sich dabei rotbraun. Nach weiterem 15minütigem Rühren gibt man bei –65 °C 18,6 g (0,1 Mol) 2,2-Dimethyl-3-formyl-cyclopropancarbonsäureäthylester zu. Man lässt dann die Reaktionsmischung auf Raumtemperatur kommen und rührt noch 3 Stunden bei 25 °C nach. Der Reaktionsansatz wird dann in 2 l Wasser gegossen und mit 600 ml Äther extrahiert. Die Ätherphase wird über Natriumsulfat getrocknet, das Lösungsmittel im Vakuum abgezogen und der ölige Rückstand bei 150–155 °C/2 Torr destilliert. Man erhält in 54,3%iger Ausbeute 2,2-Dimethyl-3-(2-chlor-2-p-chlor-phenyl-vinyl)-cyclopropancarbonsäureäthylester.

Die im oben genannten Beispiel dargestellten Cyclopropancarbonsäureäthylester können nach bekannten Verfahren sauer oder alkalisch zu den entsprechenden Säuren versteift werden. Diese können nach ebenfalls bekannten Verfahren in die entsprechenden Salze (z. B. Alkali- oder Ammoniumsalze) überführt werden.

**Patentansprüche**

1. Pentafluorbenzyloxycarbonylderivate der Formel (I)

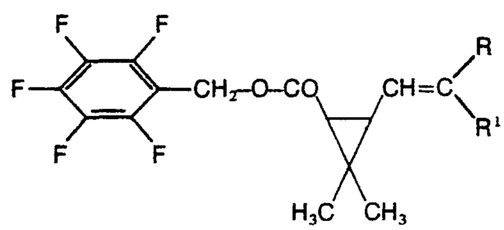

(I)

in welcher
R für Wasserstoff oder Halogen steht und
$R^1$ für gegebenenfalls substituiertes Phenyl steht, oder
worin R und $R^1$ gemeinsam für eine Alkylenkette mit mindestens zwei Kohlenstoffatomen stehen.

2. Verfahren zur Herstellung der Pentafluorbenzyloxycarbonylderivate gemäss Anspruch 1, dadurch gekennzeichnet, dass man Carbonsäuren der Formel (II)

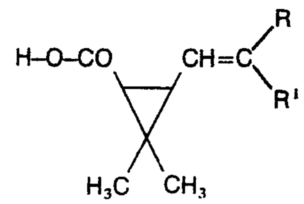

(II)

in welcher
R und $R^1$ die oben angegebene Bedeutung haben, oder deren Salze mit Pentafluorbenzylhalogeniden, gegebenenfalls in Gegenwart eines Säureakzeptors und gegebenenfalls in Gegenwart eines inerten Lösungs- oder Verdünnungsmittels umsetzt.

3. Insektizide und akarizide Mittel, gekennzeichnet durch einen Gehalt an mindestens einem Pentafluorbenzyloxycarbonylderivat der Formel (I) gemäss Anspruch 1.

4. Verfahren zur Bekämpfung von Insekten oder Spinnentieren, dadurch gekennzeichnet, dass man Pentafluorbenzyloxycarbonylderivate der Formel (I) gemäss Anspruch 1 auf Insekten oder Spinnentiere und/oder ihren Lebensraum einwirken lässt.

**Claims**

1. Pentafluorobenzyloxycarbonyl derivatives of the formula (I)

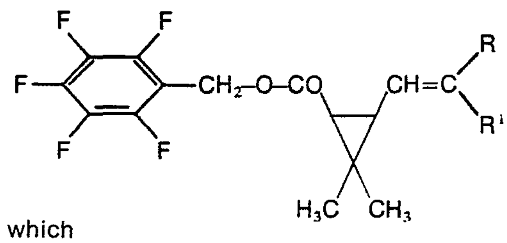

(I)

in which
R represents hydrogen or halogen and
$R^1$ represents optionally substituted phenyl, or wherein
R and $R^1$ together represent an alkylene chain with at least two carbon atoms.

2. Process for the preparation of the pentafluorobenzyloxycarbonyl derivatives according to Claim 1, characterised in that carboxylic acids of the formula (II)

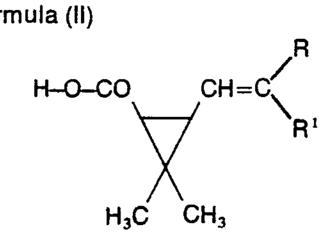

(II)

in which
R and $R^1$ have the meaning indicated above, or salts thereof are reacted with pentafluorobenzyl halides, optionally in the presence of an acid acceptor and optionally in the presence of an inert solvent or diluent.

3. Insecticidal and acaricidal agents, characterised in that they contain at least one pentafluorobenzyloxycarbonyl derivative of the formula (I) according to Claim 1.

4. Process for combating insects or arachnidae, characterised in that pentafluorobenzyloxycarbonyl derivatives of the formula (I) according to Claim 1 are allowed to act on insects or arachnidae and /or their environment.

## Revendications

1. Dérivés pentafluorobenzyloxycarbonylés de formule I

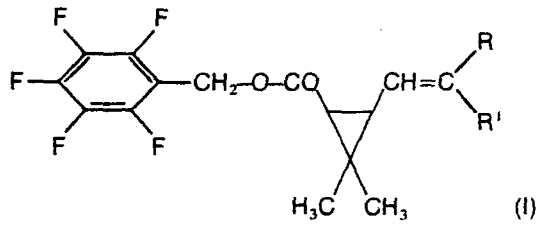

(I)

dans laquelle
R représente l'hydrogène ou un halogène et $R^1$ représente un groupe phényle éventuellement substitué, ou bien R et $R^1$ forment ensemble une chaîne alkylène à au moins 2 atomes de carbone.

2. Procédé de préparation des dérivés pentafluorobenzyloxycarbonylés selon la revendication 1, caractérisé en ce que l'on fait réagir des acides carboxyliques de formule II

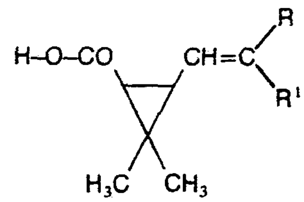

(II)

dans laquelle R et $R^1$ ont les significations indiquées ci-dessus, ou leurs sels, avec des halogénures de pentafluorobenzyle, éventuellement en présence d'un accepteur d'acide et le cas échéant en présence d'un solvant ou diluant inerte.

3. Produit insecticide et acaricide caractérisé en ce qu'il contient au moins un dérivé pentafluorobenzyloxycarbonylé de formule I selon la revendication1.

4. Procédé pour combattre les insectes ou les acariens, caractérisé en ce que l'on fait agir des dérivés pentafluorobenzyloxycarbonylés de formule I selon la revendication 1 sur les insectes ou les acariens et /ou leur espace vital.